# EUROPÄISCHE PATENTSCHRIFT

(11) **EP 2 281 794 B1**
(45) Veröffentlichungstag und Bekanntmachung des Hinweises auf die Patenterteilung: **14.10.2015**
(21) Anmeldenummer: 09167480.4
(22) Anmeldetag: 07.08.2009
(51) Int. Cl.: C07C 29/38, C07C 29/74, C07C 29/80, C07C 31/22, C07C 29/94

(54) **Verfahren zur Farbzahlverbesserung von Trimethylolpropan**
Method for improving the number of colours of trimethylolpropane
Procédé d'amélioration de la quantité de couleur de triméthylolpropane

(43) Veröffentlichungstag der Anmeldung: 09.02.2011
(73) Patentinhaber: LANXESS Deutschland GmbH, 50569 Köln (DE)
(72) Erfinder: Friederich, Michael, 47800, Krefeld (DE); Gerriets, Hans-Dieter, 47239, Duisburg (DE); Notheis, Ulrich, 41539, Dormagen (DE)

(56) Entgegenhaltungen:
- DE-A1- 1 493 048
- DE-A1- 10 164 264
- US-A- 4 514 578
- US-A- 5 603 835

## Beschreibung

Die vorliegende Erfindung betrifft ein Verfahren zur Herstellung von Trimethylolpropan mit geringer Farbzahl durch Aufarbeitung einer gemäß anorganischem Cannizarro-Verfahren erhaltenen, rohen Reaktionslösung unter Einhaltung genau definierter pH-Werte.

Trimethylolpropan, im folgenden TMP genannt, findet breite industrielle Verwendung zur Herstellung von Polyestern, Polyurethanen, Polyethern, Schaumkunststoffen, Weichmachern, Alkydharzen, Schutzanstrichen, Schmiermitteln, Appreturen und Elastomeren. Ferner substituiert es Glycerin in einigen industriellen Anwendungen.

In technischem Maßstab wird TMP beispielsweise durch das sogenannte Cannizzaro-Verfahren hergestellt, das in einem ersten Schritt eine Aldolkondensation von Butanal und Formaldehyd und in einem zweiten Schritt eine gekreuzte Cannizzaro-Reaktion zwischen dem Aldolkondensationsprodukt des ersten Schrittes und Formaldehyd umfasst. Wenn das Verfahren unter Verbrauch stöchiometrischer Mengen einer anorganischen Base wie beispielsweise Natrium- oder Calciumhydroxid verläuft wird es auch als anorganisches Cannizarro-Verfahren bezeichnet. Das im ersten Schritt als Aldolkondensationsprodukt gebildete Dimethylolbutanal reagiert dabei in der zweiten Stufe mit dem überschüssigen Formaldehyd in einer Disproportionierungsreaktion zu TMP und je nach eingesetzter Base, dem entsprechenden Formiat, wie zum Beispiel Natrium- oder Calciumformiat.

Kommerziell erhältliche TMP-Qualitäten weisen üblicherweise eine Färbung auf, die durch das Vorliegen von Verunreinigungen verursacht wird. Bei einigen Anwendungen, wie zum Beispiel die Herstellung von besonders transparenten Polyestern oder bestimmten Lackrohstoffen, ist diese Färbung jedoch störend. In der Literatur ist eine Vielzahl verschiedener Aufarbeitungsverfahren beschrieben, mit denen eine Farbzahlverbesserung von TMP erreicht werden soll.

Die Aufarbeitung TMP enthaltenden Reaktionsmischungen, wie sie nach dem Cannizzarro-Verfahren erhalten werden können, umfasst dabei im Allgemeinen eine Neutralisation der in der Reaktionsmischung verbliebenen Restbase. Dieser Neutralisation schließen sich typischerweise Schritte zur Aufkonzentrierung sowie der Abtrennung anorganischer Koppelprodukte und organischer Nebenprodukte an, wobei letztere häufig destillativ abgetrennt werden.

US 3,097,245 beschreibt ein Verfahren zur Herstellung von TMP mit einer APHA-Farbzahl zwischen 50 und 200. Diese Farbzahl wird dabei durch das Einhalten bestimmter Reaktionsbedingungen bei der Cannizarro-Reaktion hinsichtlich Temperatur, Reaktionszeit, pH-Wert und Konzentration der Ausgangsverbindungen erreicht. Weiterhin schließt sich an der Reaktion die Behandlung der erhaltenen rohen TMP-Lösung mit einem Ionenaustauscherharz an.

US 5,603,835 offenbart ein Verfahren zur Herstellung von TMP mit APHA-Farbzahlen von weniger als 100 durch extraktive Nachbehandlung von rohen TMP-Lösungen mit einem Ether oder einem Ester.

Die beiden vorstehend beschriebenen Verfahren zur TMP-Farbzahlverbesserung weisen den Nachteil auf, dass sie technisch aufwändig sind, da bestimmte Bedingungen genau eingehalten werden müssen und zusätzlich den Einsatz eines Ionenaustauscherharzes bzw. die Einführung mindestens eines Lösungsmittels erfordern.

SU-A 125552 beschreibt eine Hydrierung zur Reinigung von TMP, das nach dem Cannizzaro-Verfahren hergestellt wurde. Man erhält durch Hydrierung an Nickel-, Zink-, Molybdän- oder Kupferkatalysatoren TMP mit einem Gehalt von ca. 98 % nach Destillation. Es wird erwähnt, dass das erhaltene TMP farblos ist, wobei jedoch keine Farbzahl angegeben wird. Es hat sich jedoch in der Praxis gezeigt, dass die mit diesem Verfahren erhältliche Farbzahl für viele Zwecke nicht ausreichend ist.

Ein weiteres Verfahren zur Farbzahlverbesserung von TMP durch Hydrierung ist in der Anmeldung DE 199 63 442 A beschrieben. TMP wird hierbei nach der Herstellung destillativ gereinigt und anschließend vorzugsweise mit einem heterogenen Katalysator unter Wasserstoffdruck behandelt. Die durch Hydrierung erreichte Farbzahl kann durch vorheriges mehrmaliges Destillieren weiter verbessert werden.

Nachteilig bei allen Hydrierverfahren zur Farbzahlverbesserung ist jedoch der hohe apparative Aufwand sowie eine Qualitätsminderung des erhaltenen TMP durch Katalysatorabrieb. Ferner vermindern die Kosten für den Katalysator die wirtschaftliche Effizienz.

In der DE 100 29 055 A ist eine Verfahren zur Farbzahlverbesserung von TMP beschrieben, bei dem destillativ vorgereinigtes TMP mit einer Reinheit von vorzugsweise > 95 % einer Temperung, vorzugsweise bei Temperaturen von 160 bis 240 °C unterworfen wird und das TMP anschließend erneut, vorzugsweise durch Destillation, gereinigt wird. Durch den Temperschritt werden die farbzahlgebenden Nebenkomponenten in höher siedende, schwerflüchtige Komponenten überführt. Nachteilig bei diesem Verfahren ist jedoch, dass sich an die Temperung erneut ein Reinigungsschritt anschließen muss, beispielsweise eine Destillation, bzw. dieser Reinigungsschritt mit der Temperung gekoppelt werden muss, um diese höher siedenden Nebenkomponenten zu entfernen und ein TMP mit niedriger Farbzahl zu erhalten.

DE1493048 OS beschreibt die Aufarbeitung mittels eines Dünnschichtverdampfers, über den das rohe TMP destilliert wird. Das rohe TMP wird zunächst neutralisiert, dann zentrifugiert und über einen Schnellverdampfer geführt und anschließend vor der finalen Aufreinigung auf einen pH-Wert von ungefähr 4 bis 8 vorzugsweise 5 bis 6 gebracht. Die Farbzahl des erhaltenen TMPs kann 0 APHA erreichen. Nachteilig an diesem Verfahren ist, dass an zwei Stellen im Prozess Säure zugegeben werden muss. Welche Säure zum Neutralisieren und pH-Stellen verwendet wird, ist nicht abgegeben. DE 10164264 A beschreibt ein Verfahren zur Herstellung eines TMP mit geringer APHA-Zahl, bei dem n-Butyraldehyd mit Formaldehyd in Gegenwart von anorganischer Base zu TMP-haltigen Reaktionsmischungen umgesetzt wird, aus diesen Reaktionsmischungen Wasser und anorganische Salze zumindest teilweise entfernt werden und das gewonnene rohe TMP nach zumindest teilweiser Abtrennung von Schwersiedern und Nichtsiedern durch Destillation in eine oder mehrere Leichtsiederfraktionen, eine oder mehrere überwiegend TMP-haltige Mittelsiederfraktionen und eine oder mehrere Schwersieder- und/oder Nichtsiederfraktionen getrennt wird.

Aus der US 4,514,578 ist ein Verfahren zur Herstellung von TMP durch Umsetzung von n-Butyraldehyd und wässrigem Formaldehyd in Gegenwart von alkalischen Kondensationsmitteln bekannt, be dem ein pH Wert zwischen 5,5 bis 6,5 in der Reaktionsmischung nach Beendigung der Reaktion durch Verwendung von Ameisensäure, Essigsäure, Schwefelsäure und/oder Phosphorsäure eingestellt wird.

Den Beispielen der DE 10164264 A ist zur entnehmen, dass nach der Kondensationsreaktion die Reaktionsmischung mit Ameisensäure auf pH 6 eingestellt wird, die Neutralisation wird nicht diskutiert und in keinen Zusammenhang mit der Farbzahl gebracht. Anschließend wird die Reaktionsmischung aufkonzentriert, Calciumformiat abfiltriert, nochmals aufkonzentriert und die dann erhaltene rohe TMP Mischung über einen Dünnschichtverdampfer destilliert. Das Kopfprodukt des Dünnschichtverdampfers hat einen Gehalt Von 94,9% und eine APHA-Farbzahl von 148 und wird in weiteren Destillationsschritten auf eine Reinheit von 99,4% bzw 99,1% und eine APHA-Farbzahl von 13 bzw 8 gebracht. Im Vergleichsbeispiel wird eine Reinheit von 99,3% und eine APHA-Farbzahl von 22 erhalten. Dieses Verfahren hat, obwohl es TMP mit guter Farbzahl liefert, den Nachteil, dass dazu in der Destillation hohe Rücklaufverhältnisse von 48 angewendet werden.

Es bestand demnach weiterhin die Aufgabe, ein effizientes Verfahren zur Herstellung von TMP mit niedriger Farbzahl bereitzustellen, das die Nachteile des Standes der Technik überwindet.

Es wurde nun ein Verfahren zur Herstellung von TMP durch Umsetzung von Butyraldehyd mit Formaldehyd in Gegenwart einer anorganischen Base zu einem TMP-haltigen Reaktionsgemisch und anschließender Aufarbeitung des TMP-haltigen Reaktionsgemisches gefunden, das dadurch gekennzeichnet ist, dass die Aufarbeitung des TMP-haltigen Reaktionsgemisches die Einstellung des pH-Wertes des TMP-haltigen Reaktionsgemisches auf einen Welt von 4,50 bis 5,45 bezogen auf eine Temperatur von 50°C umfasst, wobei zur Einstellung des pH-Wertes alsorganischen Carbonsäure Ameisensäure verwendet wird und die Aufarbeitung weiterhin folgende Schritte umfasst:
a) Entfernung einer Teilmenge von Wasser und gegebenenfalls Formaldehyd aus dem TMP enthaltenden, eingestellten Reaktionsgemisch
b) Abtrennung von Calciumformiat aus dem TMP enthaltenden, eingestellten Reaktionsgemisch zum Erhalt einer TMP enthaltenden Rohlösung.
c) Abtrennung von Schwersiedern aus der TMP enthaltenden Rohlösung zum Erhalt eines rohen. TMP
d) Destillation des gemäß Schritt c) erhaltenen rohen TMP zum Erhalt von TMP mit geringer Farbzahl.

Der Rahmen der Erfindung umfasst alle beliebigen und möglichen Kombinationen der im Folgenden aufgeführten, allgemeinen oder in Vorzugsbereichen genannten Komponenten, Wertebereiche bzw. Verfahrensparameter.

Das molare Verhältnis von Formaldehyd zu Butyraldehyd bei der Umsetzung beträgt beilspielsweise 2,5 bis 6,0, bevorzugt 3,0 bis 5,0, besonders bevorzugt 3,0 bis 4,0 und ganz besonders bevorzugt 3,1 bis 3,5.

Das molare Verhältnis von Baseäquivalenten aus den anorganischen Basen zu Butyraldehyd bei der Umsetzung beträgt beispielsweise 0,8 bis 2,5, bevorzugt 1,0 bis 2,0, besonders bevorzugt 1,0 bis 1,4 und ganz besonders bevorzugt 1,05 bis 1,2.

Als anorganische Basen kommen vorzugsweise Hydroxide oder Carbonate von Alkali- oder Erdalkalimetallen zum Einsatz, besonders bevorzugt sind Natrium- und Calciumhydroxid, wobei Calciumhydroxid noch weiter bevorzugt ist. Bei Einsatz von Calciumhydroxid kann dieses beispielsweise als technische Ware mit einer Reinheit von mehr als 80%, bevorzugt 90%, besonders bevorzugt 95% eingesetzt werden.

Demzufolge kann das molare Verhältnis von Calciumhydroxid zu Butyraldehyd beispielsweise 0,4 bis 1,25, bevorzugt 0,5 bis 1,0, besonders bevorzugt 0,5 bis 0,7 und ganz besonders bevorzugt 0,525 bis 0,6 liegen.

Formaldehyd wird vorzugsweise als wässrige Lösung mit einem Gehalt von 10 bis 50 Gew.-% eingesetzt. In einer bevorzugten Variante wird Formaldehyd mit einer Gehalt von 25 bis 40 Gew.-% eingesetzt. Der Reaktionsmischung kann zusätzliches Wasser zugesetzt werden, was die Selektivität der Umsetzung verbessert, jedoch den Aufwand in der Aufarbeitung wegen der höheren Wassermengen erhöht.

Bei der Umsetzung kann weiterhin zumindest eine Verbindung zugesetzt werden, die in der Lage ist, Formosereaktionen zu vermeiden oder zumindest zu unterdrücken. Geeignete Verbindungen sind dem Fachmann gekannt und beispielsweise in US 2,186,272, US 2,329,515, EP 510 375 A, DE OS 41 23 062 und DE OS 41 26 730 beschrielien. Sie umfassen beispielweise Metallsalze von Kupfer, Eisen, Mangan, Chrom, Nickel, Kobalt, Silber, Platin, Blei, Molybdän, Wolfram, Wismuth Vanadium, Zirkonium, Titan, Niob, Hafnium gegebenenfalls in Kombination mit einer Begasung mit Luft oder Sauerstoff oder Borverbindungen. Bevorzugt sind Eisensalze wie Eisen(II)sulfat und seine Hydrate, sowie Eisen(III)sulfat und seine Hydrate. Die Menge der eingesetzten Verbindungen kann dabei beispielsweise 50 bis 1000 ppm bezogen auf die Gesamtmenge des Reaktionsgemisches betragen.

Zur Umsetzung wird bevorzugt zumindest ein Teil des Formaldehyds und der anorganischen Base vorgelegt und Butyraldehyd sowie gegebenenfalls Anteile des Formaldehyds über einen Zeitraum von 3 bis 180 Minuten, bevorzugt 15 bis 120 Minuten, besonders bevorzugt 20 bis 60 Minuten zugegeben.

Die Temperatur bei der Umsetzung kann beispielsweise 20 bis 90°C, bevorzugt 20 bis 60°C betragen.

In einer bevorzugten Variante lässt man die Temperatur während der Zugabe von Butyraldehyd kontrolliert ansteigen, beispielsweise im Wesentlichen linear von 20 auf 60°C,

Nach Beendigung der Zugabe wird beispielsweise 5 bis 180 Minuten, bevorzugt 30 bis 90 Minuten bei der Endtemperatur nachgerührt.

In einer anderen bevorzugten Fahrweise wird die Umsetzung kontinuierlich z.B in einem Rohrreaktor, einer Kesselkaskade oder einem Plattenwärmetauscher ausgeführt.

Die nach der Umsetzung erhaltenen TMP-haltigen Reaktionsgemische weisen typischerweise einen pH-Wert zwischen 9,0 und 11,0 auf.

Die erhaltenen TMP-haltigen Reaktionsgemische werden dann auf einen pH-Wert von 4,50 bis 5,45 bezogen auf eine Temperatur von 50°C durch Ameisensäure eingestellt.

Überraschenderweise kann die Farbzahl des TMPs, der in der folgenden Aufarbeitung durch den eingestellten pH-Wert in einfacher Weise beeinflusst werden. Dies geschieht in der Weise, dass die Farbzahl typischerweise umso geringer wird je niedriger der pH-Wert im genannten Bereich wird.

Allerdings können be niedrigeren pH-Werten in Abhängigkeit von der Temperatur und der Verweilzeit in der weiteren Aufarbeitung auch Nebenreaktion wie die Bildung von TMP-Formiat auftreten was letztendlich die Ausbeuten an TMP verringert. Weiterhin führt ein zu geringer pH-Wert zu einer Zersetzung von Restformaldehyd im Roh-TMP und damit zum Schäumen in der ersten Destillation, die einen stabilen Betrieb der Anlage verhindert.

Es ist für den Fachmann ein Leichtes, in einem Vorversuch in Abhängigkeit der Parameter, die für die Vorangegangene Umsetzung gewählt wurden, zu ermitteln bei welchem pH-Wert in der Neutralisierung eine ausgezeichnete Farbzahl bei gleichzeitig hoher Ausbeute erzielt werden kann.

Die sich an die Einstellung des pH-Wertes anschließende, weitere Aufarbeitung kann in an sich bekannter Weise erfolgen. Dabei kann die Aufarbeitung batchweise oder kontinuierlich erfolgen, wobei eine kontinuierliche weitere Aufarbeitung bevorzugt ist.

In einer weiteren bevorzugten Ausführungsform umfasst die sich an die erfindungsgemäße pH-Wert-Einstellung anschließende, weitere Aufarbeitung die Schritte a) bis d) in der genannten Reihenfolge.:
Schritt a) erfolgt vorzugsweise durch Destillation beispielsweise in einer mehrstufigen Fahrweise bei verschiedenen Druckniveaus. Dabei wird neben Wasser üblicherweise auch überschüssiges Förmaldehyd zumindest teilweise entfernt. Im Läufe der Entfernung von Wasser können insbesondere bei Verwendung von Calciumhydroxid als Base, anorganische Formiate ausfallen, die im Schritt b) abgetrennt werden.
Schritt b) kann beispielsweise durch Filtration, Sedimentation und Dekantieren oder Zentrifugation erfolgen, vorzugsweise durch Filtration oder Zentrifugation.

Die Temperatur bei der Abtrennung kann beispielsweise 20 bis 90°C vorzugsweise 50 bis 90°C betragen.

Gegebenenfalls können die Schritte a) und b) einfach oder mehrfach wiederhol werden.
Schritt c) erfolgt vorzugsweise durch Destillation. In einer bevorzugten Ausführunsform erfolgt die Destillation über Dünnschicht- oder Kurzwegverdampfer oder andere geeignete Vorrichtungen, die eine kurze Verweilzeit ermöglichen. Gemäß Schritt c) werden Schwersieder aus der gemäß Schritt b) erhaltenen TMP-Rohlösung abgetrennt, wobei unter Schwersiedern solche Verbindungen zu verstehen sind, die höher als TMP sieden oder keinen merklichen Dampfdruck besitzen. Beispiele für Schwersieder sind Di-TMP und Bis-TMP-Formal sowie höhere Oligomere und Formale von TMP.

Typischerweise wird in Schritt c) rohes TMP mit einer Reinheit von mehr als 90 Gew.-% erhalten.
Im Schritt d) wird das gemäß Schritt c) erhaltene rohe TMP destillativ weiter gereinigt. Dies kann in einer bevorzugten Ausführungsform dadurch geschehen, dass zunächst in einer ersten Kolonne Leichtsieder vom TMP abgetrennt werden. Leichtsieder sind in diesem Zusammenhang Verbindungen mit einem niedrigeren Siedepunkt als TMP, wie beispielsweise 2-Hydroxymethyl-1-butanol, 1-Methoxy-2,2-di(hydroxymethyl)-butan und 1-[(Methoxy)methyloxy]-2,2-di-(hydroxymethyl)-butan. TMP fällt im Sumpf dieser Kolonne an und wird anschließend in einer weiteren Kolonne über Kopf destilliert. Schritt d) kann weiterhin in einem einzigen Destillationsschritt durch thermisch gekoppelte Kolonnen durchgeführt werden. Bekannte Alternativen dazu sind auch Seitenabzugskolonnen mit oder ohne Trenneinrichtungen wie z.B. einer Trennwand, in der das reine TMP als Seitenstrom entnommen werden kann.

In einer alternativen Ausführungsform können auch die Schritte c) und d) gleichzeitig in einer Destillation durchgeführt werden, wobei die für Schritt c) beschriebenen Vorrichtungen in gleicher Weise eingesetzt werden können.

Auf erfindungsgemäße Weise wird TMP mit geringer Farbzahl und höher Reinheit erhalten.

### Beispiele

### Allgemeines:

Die APHA-Farbzahlen [Einstufung der Farbe nach der Platin Cobalt Skala, vgl. DIN ISO 6271] wurden mit einem Photometer Lico 300 der Fa. Dr. Lange bestimmt. Dazu wurden jeweils 4 bis 5 g TMP in eine 11 mm Rundküvette gefüllt, die Rundküvette mit einem Silikonstopfen verschlossen, bei 100°C aufgeschmolzen und vermessen. Für jede Probe wurden zwei Messungen durchgeführt und die Werte gemittelt.

### Beispiel 1:

### Einfluss der pH-Einstellung auf die Farbzahl des Rohprodukts nach der Schwersiederabtrennung

Batchweise wurden 3,15 Mol-Äquivalente 32 Gew.-%iges Formaldehyd mit Wasser verdünnt und bei Raumtemperatur mit 0,56 Mol-Äquivalenten Calciumhydroxid versetzt. In 50 Minuten wurden 1,00 Mol-Äquivalent Butyraldehyd in den Kessel einlaufen gelassen und die Mischung dabei auf 50°C erwärmt. Die Butyraldehydmenge bezogen auf die Gesamtmasse betrug 10 Gew.-%, die Endkonzentration an Trimethylolpropan betrug 15 bis 15,3 Gew.-%. Nach der Reaktion rührte man 40 Minuten bei 50°C nach und stellte den pH-Wert auf die in der Tabelle angegebenen Werte ein. Die Reaktionsmischung wurde anschließend kontinuierlich in einem dreistufigen Verdampfer bei Temperaturen von 50 bis 80°C und Drucken von 130 bis 260 mbar auf einen Restwassergehalt von 30 bis 33 Gew.-% aufkonzentriert. Danach wurde bei 75 bis 80°C ausgefallenes Calciumformiat abgetrennt und bei 80 bis 100 mbar und 100°C der Restwassergehalt auf 1,5 Gew.-% abgesenkt. Dabei ausgefallene Restfeststoffe, überwiegend Calciumformiat wurden mit einem Dekanter abgetrennt und das rohe TMP bei 10 mbar und 140 bis 150°C von restlichen Leichtsiedern befreit. Das von Leichtsiedern befreite Roh-TMP wurde in einem Kurzwegverdampfer bei 3 mbar und 130 bis140°C bis zu einem Abdampfgrad von 60 % über Kopf destilliert. Der Sumpfablauf des Kurzwegverdampfers wurde in drei parallelen Dünnschichtverdampfern (D SV) bei 5 bis10 mbar und einer Temperatur von 170°C im Kopf so weiter aufkonzentriert, dass man einen Sumpfablauf mit weniger als 10 Gew.-% Rest-TMP erhielt.

Die Rohdestillate wurden vereinigt und in einer ersten Kolonne mit einer Sumpftemperatur von 230°C, einer Kopftemperatur von 154°C bei einem Kopfdruck von 30 mbar von Leichtsiedern befreit. Der Sumpfablauf dieser Kolonne wurde in einer weiteren Kolonne bei 220°C Sumpftemperatur, und 136°C bei 12 mbar im Kopf der Kolonne über Kopf destilliert und hatte dann einen TMP-Gehalt von >99 Gew.-%.

Vom Roh-TMP nach den Dünnschichtverdampfern und der Endware nach der zweiten Destillationskolonne wurden Proben gezogen und die APHA-Farbzahl bestimmt.

| **pH-Wert in der Reaktion** | **APHA Kopfprodukt DSV 1** | **APHA Kopfprodukt DSV 2** | **APHA Kopfprodukt DSV 3** | **APHA Kopfprodukt zweite Kolonne** |
|---|---|---|---|---|
| 5,25 | 110 | 120 | 150 | 7 |
| 5,30 | 130 | 150 | 190 | 9 |
| 5,45 | 170 | 180 | 260 | 13 |
| | | | | |

| (nicht erfindungsgemäß) | | | | |
|---|---|---|---|---|
| **pH-Wert in der Reaktion** | **APHA Kopfprodukt DSV 1** | **APHA Kopfprodukt DSV 2** | **APHA Kopfprodukt DSV 3** | **APHA Kopfprodukt zweite Kolonne** |
| 5,50 | 200 | 200 | | 15 |

Die Beispiele zeigen, dass bereits in der Schwersiederabtrennung, die über einen Dünnschichtverdampfer mit lediglich einer Trennstufe erfolgt, mit niedrigerem pH-Wert ein deutlich farbarmes Rohprodukt erhalten wird. Die APHA-Farbzahl des TMP nach der letzten Destillation vor der Konfektionierung wurde unter Berücksichtigung der Verweilzeit bestimmt. Das TMP hatte in allen Fällen eine Reinheit zwischen 99,4% und 99,8 Gew.-%.

Die Versuche zeigen, dass bereits geringe Absenkungen des pH-Wertes deutliche Verbesserungen der Farbzahl zur Folge haben.

## Patentansprüche

1. Verfahren zur Herstellung von TMP durch Umsetzung von Butyraldehyd mit Formaldehyd in Gegenwart einer anorganische Base zu einem TMP-haltigen Reaktionsgemisch und anschließender Aufarbeitung des TMP-haltigen Reaktionsgemisches, **dadurch gekennzeichnet, dass** die Aufarbeitung die Einstellung des pH-Wertes des TMP-haltigen Reaktionsgemisches auf einen Wert von 4,50 bis 5,45 bezogen auf eine Temperatur von 50°C umfasst, wobei zur Einstellung des pH-Wertes als organische Carbonsäure Ameisensäure verwendet wird, und die Aufarbeitung weiterhin zumindest folgende Schritte umfasst:
a) Entfernung von Wasser und gegebenenfalls Formaldehyd aus dem TMP enthältenden, eingestellten Reaktionsgemisch
b) Abtrennung von festen Bestandteilen des TMP enthaltenden, eingestellten Reaktionsgemisches zum Erhalt einer TMP enthaltenden Rohlösung
c) Abtrennung von Schwersiedern aus der TMP enthältenden Rohlösung zum Erhält eines rohen TMP
d) Destillation des gemäß Schritt c) erhaltenen rohen TMP zum Erhalt von TMP mit geringer Farbzahl.

2. Verfahren nach Anspruch 1, **dadurch gekennzeichnet, dass** als anorgganische Basen Hydroxide oder Carbonate von Alkali- oder Erdalkalimetallen eingesetzt werden.

3. Verfahren nach Anspruch 1 oder 2, **dadurch gekennzeichnet, dass** das erhaltene TMP-haltige Reaktionsgemisch auf einen pH-Wert von 5,00 bis 5,45 bezogen auf eine Temperatur von 50°C eingestellt wird.

4. Verfahren nach einem der Ansprüche 1 bis 3, **dadurch gekennzeichnet, dass** das erhaltene TMP-haltige Reaktionsgemisch auf einen pH-Wert von 5,25 bis 5,45 bezogen auf eine Temperatur von 50°C eingestellt wird.

5. Verfahren nach Anspruch 4, **dadurch gekennzeichnet, dass** die Schritte a) und b) zumindest einmal wiederholt werden.

6. Verfahren nach Anspruch 4 oder 5, **dadurch gekennzeichnet, dass** die Schritte c) und d) gleichzeitig durchgeführt werden.

7. Verfahren nach einem oder mehreren der Ansprüche 1 bis 6, **dadurch gekennzeichnet, dass** anschließend das TMP zur Herstellung von Polyestern, Polyurethanen, Polyethern, Schaumkunststoffen, Weichmachern, Alkydharzen, Schutzanstrichen, Schmiermitteln, Appreturen und Elastomeren eingesetzt wird.

## Claims

1. Process for preparing TMP by reaction of butyraldehyde with formaldehyde in the presence of an inorganic base to give a TMP-containing reaction mixture and subsequent work-up of the TMP-containing reaction mixture, **characterized in that** the work-up comprises setting the pH of the TMP-containing reaction mixture to a value of from 4.50 to 5.45 based on a temperature of 50°C, wherein formic acid is used as organic carboxylic acid for setting the pH, and the work-up further comprises at least the following steps:
a) removal of water and optionally formaldehyde from the TMP-containing, adjusted reaction mixture,
b) separation of solid constituents from the TMP-containing, adjusted reaction mixture to give a TMP-containing crude solution,
c) separation of high boilers from the TMP-containing crude solution to give a crude TMP,
d) distillation of the crude TMP obtained according to step c) to give TMP having a low color number.

2. Process according to Claim 1, **characterized in that** hydroxides or carbonates of alkali metals or alkaline earth metals are used as inorganic bases.

3. Process according to Claim 1 or 2, **characterized in that** the TMP-containing reaction mixture obtained is set to a pH of 5.00 to 5.45, based on a temperature of 50°C.

4. Process according to any of Claims 1 to 3, **characterized in that** the TMP-containing reaction mixture obtained is set to a pH of 5.25 to 5.45, based on a temperature of 50°C.

5. Process according to Claim 4, **characterized in that** steps a) and b) are repeated at least once.

6. Process according to Claim 4 or 5, **characterized in that** steps c) and d) are carried out simultaneously.

7. Process according to one or more of Claims 1 to 6, **characterized in that** the TMP is subsequently used for production of polyesters, polyurethanes, polyethers, polymer foams, plasticizers, alkyd resins, protective paints, lubricants, finishes and elastomers.

## Revendications

1. Procédé pour la préparation de TMP par transformation de butyraldéhyde avec du formaldéhyde en présence d'une base inorganique en un mélange réactionnel contenant du TMP et traitement consécutif du mélange réactionnel contenant du TMP, **caractérisé en ce que** le traitement comprend l'ajustement du pH du mélange réactionnel contenant du TMP à une valeur de 4,50 à 5,45, rapportée à une température de 50°C, l'acide formique étant utilisé comme acide carboxylique organique pour l'ajustement du pH, et le traitement comprenant en plus au moins les étapes suivantes :
a) élimination de l'eau et le cas échéant du formaldéhyde du mélange réactionnel ajusté contenant du TMP,
b) séparation de constituants solides du mélange réactionnel ajusté contenant du TMP pour obtenir une solution brute contenant du TMP
c) séparation des produits à point d'ébullition élevé de la solution brute contenant du TMP pour obtenir un TMP brut
d) distillation du TMP brut obtenu selon l'étape c) pour obtenir du TMP présentant un faible indice de couleur.

2. Procédé selon la revendication 1, **caractérisé en ce qu'**on utilise comme bases inorganiques des hydroxydes ou des carbonates de métaux alcalins ou alcalino-terreux.

3. Procédé selon la revendication 1 ou 2, **caractérisé en ce que** le mélange réactionnel contenant du TMP obtenu est ajusté à une valeur de pH de 5,00 à 5,45, rapportée à une température de 50°C.

4. Procédé selon l'une quelconque des revendications 1 à 3, **caractérisé en ce que** le mélange réactionnel contenant du TMP obtenu est ajusté à une valeur de pH de 5,25 à 5,45, rapportée à une température de 50°C.

5. Procédé selon la revendication 4, **caractérisé en ce que** les étapes a) et b) sont répétées au moins une fois.

6. Procédé selon la revendication 4 ou 5, **caractérisé en ce que** les étapes c) et d) sont réalisées simultanément.

7. Procédé selon l'une quelconque ou plusieurs des revendications 1 à 6, **caractérisé en ce qu'**ensuite le TMP est utilisé pour la préparation de polyesters, de polyuréthanes, de polyéthers, de matières plastiques moussées, de plastifiants, de résines alkydes, d'enduits de protection, de lubrifiants, d'apprêts et d'élastomères.
